Europäisches Patentamt

European Patent Office

Office européen des brevets

(19)

(11) Publication number : **0 188 782 B2**

# NEW EUROPEAN PATENT SPECIFICATION

(12)

(45) Date of publication of the new patent specification : **21.10.92 Bulletin 92/43**

(51) Int. Cl.⁵ : **G01N 23/04**

(21) Application number : **85116296.6**

(22) Date of filing : **19.12.85**

(54) Sectional radiography display method and apparatus.

(30) Priority : **26.12.84 JP 280613/84**

(43) Date of publication of application :
**30.07.86 Bulletin 86/31**

(45) Publication of the grant of the patent :
**04.10.89 Bulletin 89/40**

(45) Mention of the opposition decision :
**21.10.92 Bulletin 92/43**

(84) Designated Contracting States :
**DE FR GB**

(56) References cited :
EP-A- 0 098 398
DE-A- 2 503 978
DE-A- 2 613 025
FR-A- 1 515 861
GB-A- 1 564 309
GB-A- 2 137 453
PATENT ABSTRACTS OF JAPAN, vol. 3, no.
150 (E-158), 11th December 1979, page 17 E
158 & JP - A - 54 129 895
PATENT ABSTRACTS OF JAPAN, vol. 2, no.
127 (E 78), 25th October 1978, page 7689 E 78 &
JP - A - 53 94889
PATENT ABSTRACTS OF JAPAN, vol. 6, no.
168 (P-139)[1046], 2nd September 1982 & JP - A
- 57 86745
PATENT ABSTRACTS OF JAPAN, vol. 8, no.
183 (P-296)[1620], 23rd August 1984 & JP - A -
59 75139

(56) References cited :
C. Leon Partain et al., "Nuclear Magnetic
Resonance (NMR) Imaging", 1983, W.B. Saun-
ders Company, Philadelphia, U.S., pp.466-467;
Derek Shaw, "Fourier Transform N.M.R. Spec-
troscopy, 2nd Edition, 1984, Elsevier, Amster-
dam, NL

(73) Proprietor : **HITACHI, LTD.**
**6, Kanda Surugadai 4-chome**
**Chiyoda-ku, Tokyo 100 (JP)**

(72) Inventor : **Ogura, Satoshi**
**Jikyo-ryo, B-211, 6-12-1 Ayukuwa-cho**
**Hitachi-shi Ibaraki-ken (JP)**
Inventor : **Takahashi, Fuminobu**
**125, 3271, Nakane**
**Katsuta-shi Ibaraki-ken (JP)**
Inventor : **Kanamori, Takahiro**
**475, 3600, Nakane**
**Katsuta-shi Ibaraki-ken (JP)**
Inventor : **Koga, Kazunori**
**Jikyo-ryo, B-404 6-12-1, Ayukawa-cho**
**Hitachi-shi Ibaraki-ken (JP)**
Inventor : **Koike, Masahiro**
**Jikyo-ryo, B-312 6-12-1, Ayukawa-cho**
**Hitachi-shi Ibaraki-ken (JP)**
Inventor : **Itoh, Shinichi**
**Chimei-ryo, 312 3-1, Jyonan-cho**
**Hitachi-shi Ibaraki-ken (JP)**

(74) Representative : **Patentanwälte Beetz sen. -**
**Beetz jun. Timpe - Siegfried - Schmitt-Fumian**
**Steinsdorfstrasse 10**
**W-8000 München 22 (DE)**

EP 0 188 782 B2

## Description

BACKGROUND OF THE INVENTION

The present invention relates to an apparatus of the kind referred to in the preamble portion of claim 1. Such an apparatus is known from DE-A-2613025 or DE-A-2503978.

Among radiographic methods applying an X-ray CT apparatus, T-R system was used, for instance, in an early stage, in which an X-ray source and a detector were set opposite to each other with a subject held between them so as to conduct translational and rotational scanning (in the first generation), as described in "CT scanner", published in Deshikogaki Shinpo Series 9, Corona Sha. Thereafter, a T-R II system in which a plurality of detectors and a somewhat expanded X-ray source were employed for reducing the time required for radiography (the second generation), and an R-R system in which an X-ray source and a plurality of detectors were turned simultaneously around a subject (the third generation), were developed, and now, an S-R system in which detectors are set stationary on the whole circumference of a radiographing region and an X-ray source is rotated in the scanning direction while emitting X-ray beams 4 into the entire scope of the radiographing region (the fourth generation) has been put to practical use. The X-ray source stops rotation at a data sampling point set for radiographing and performs a radiographing operation at this point. It repeats the same operation along the rotational track of X-ray source thereof, and the radiographing operation is stopped when the X-ray source makes a round of the radiographing region. This system has an advantage that it enables a projection of high accuracy and high speed, since a mechanically-movable member is provided only for rotating the X-ray source. This system, however, has as disadvantage that the X-ray beam cannot be collimated sufficiently by the detectors. This is because the detectors cannot be arranged closely to each other for structural reasons, and because each of them is directed not to the X-ray source, but to the center of the radiographing region. Accordingly, the amount of detected X-rays at the position of the detector is represented by polar coordinates in which the detected amount is presented in the radial direction. The path of the X-ray beams is represented herein by a straight line connecting the position of the X-ray source and the position of the detector. Consequently, the data on detected X-rays are necessarily discrete to some degree, which results in a limited resolution of an image display.

A second disadvantage due to the fact that X-rays cannot be collimated sufficiently will be described hereunder. In general, a scintillator is used as detector. As for the detection sensitivity of the scintillator, however, only the evaluation thereof in the case when the incidence is vertical to the detecting surface of the scintillator is employed, and therefore, the evaluation of the detection sensitivity in the case when the incidence is oblique to the detecting surface of the scintillator is difficult. Moreover, the nonuniformity in the angular distribution of X-ray beams is another cause of the difficulty in the evaluation of the detection sensitivity.

Thus, it can be said that the method of X-ray radiography applying the S-R system aims a high-speed radiography, but leads to a reduction of the density of radiographic data and to a sacrifice of the detection sensitivity of each detector to some degree.

Prior art document GB-A-15 64 309 discloses a cross-sectional radiography imaging apparatus comprising a radiation source, a plurality of radiation detectors for detecting radiant rays emitted from said radiation source (and transmitted through an object to be measured and for providing output signals indicative thereof, said radiation detectors being connected together for simultaneous movement, detector driving means for moving said radiation detectors, means for computing cross-sectional imaging information on said object in response to the output signals from said radiation detectors, and means for displaying a cross-sectional image of the object in accordance with the output of said computing means.

This document further shows a sectional radiography imaging method comprising the following steps:
emitting a radiation beam on an object to be measured;
detecting emitted radioactive rays being transmitted through the object;
moving a radiation detector on a part of a circumference around the source of said radiation beam;
computing sectional display information on said object from output signals of said detector and
displaying a sectional image of the object on the basis of the results of the above computation.

From Patent Abstracts of Japan, Vol. 6, no. 168 (P-139) (1046), September 2, 1982, a multichannel X-ray detector is known which is moved together with an X-ray source in a rotating matter in synchronism with each other and in the same direction centering around an object to be irradiated to collect X-ray data on various directions in a section of the object.

SUMMARY OF THE INVENTION

An object of the present invention is to furnish an improved sectional radiography display apparatus.

This object is achieved with the apparatus, as claimed in claim 1.

According to the present invention the radiation transmission distribution has a precision being two or more times that obtained with conventional methods which can be attained at less than half the cost that is needed for conventional methods so that an improvement in the image accuracy which has not been available heretofore can be obtained by a CT apparatus and the method according to the present invention.

## BRIEF DESCRIPTION OF THE DRAWINGS

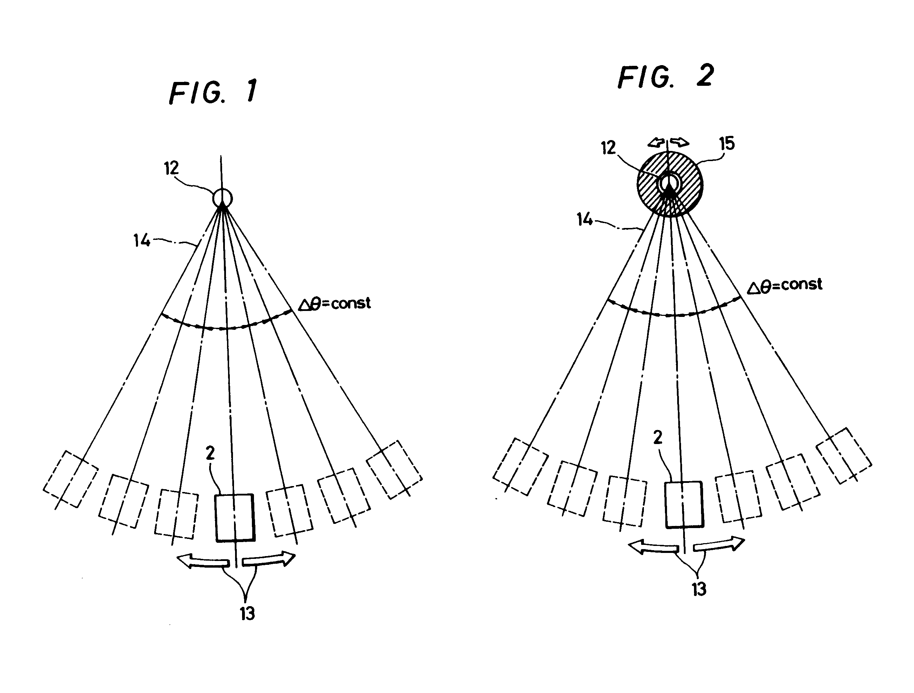

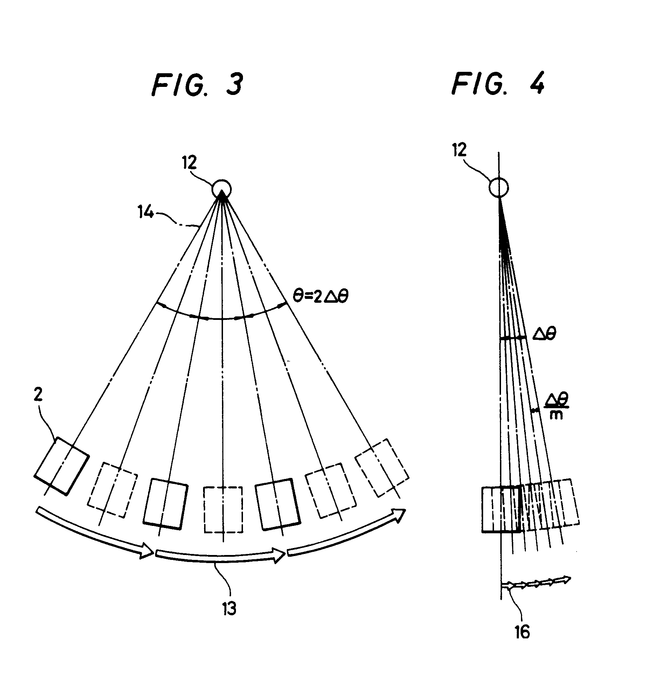

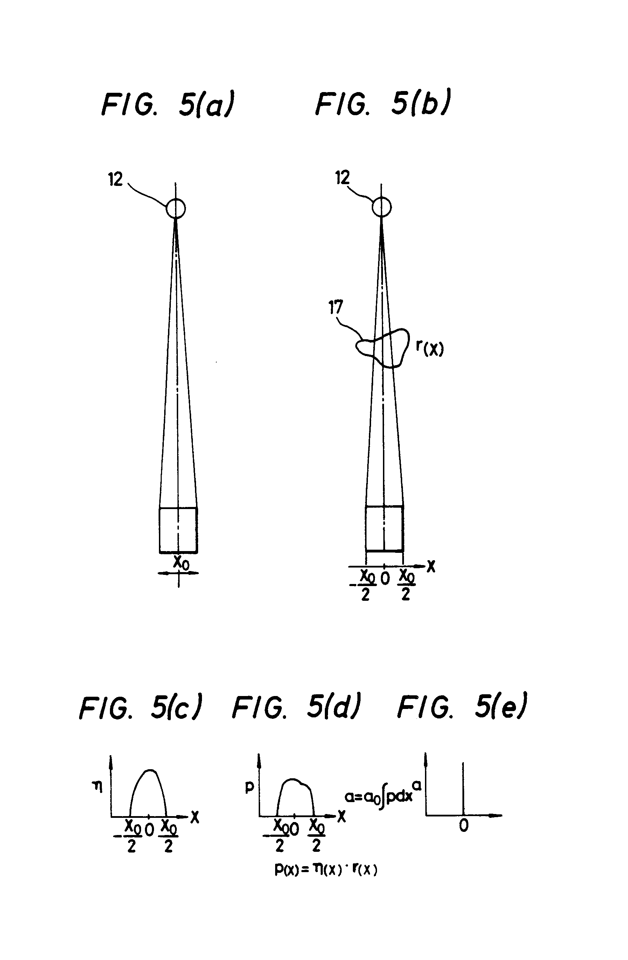

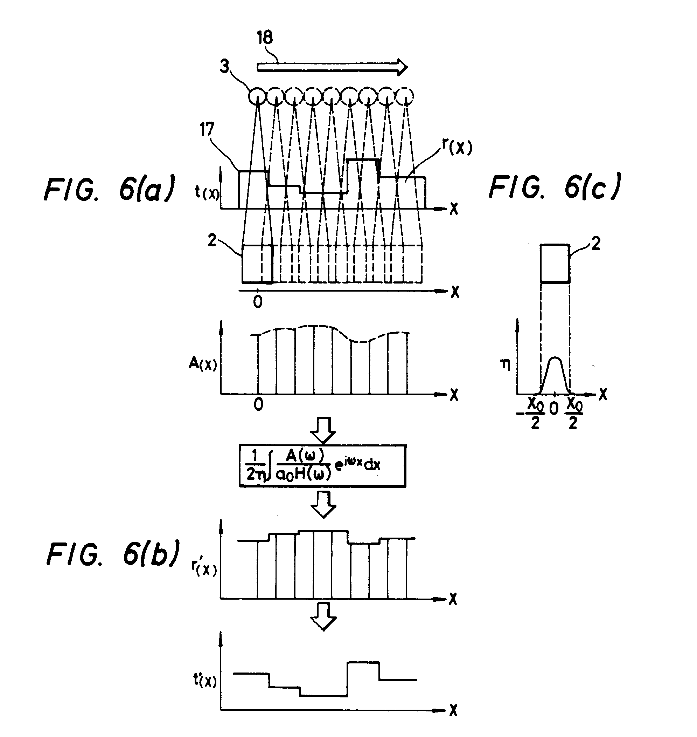

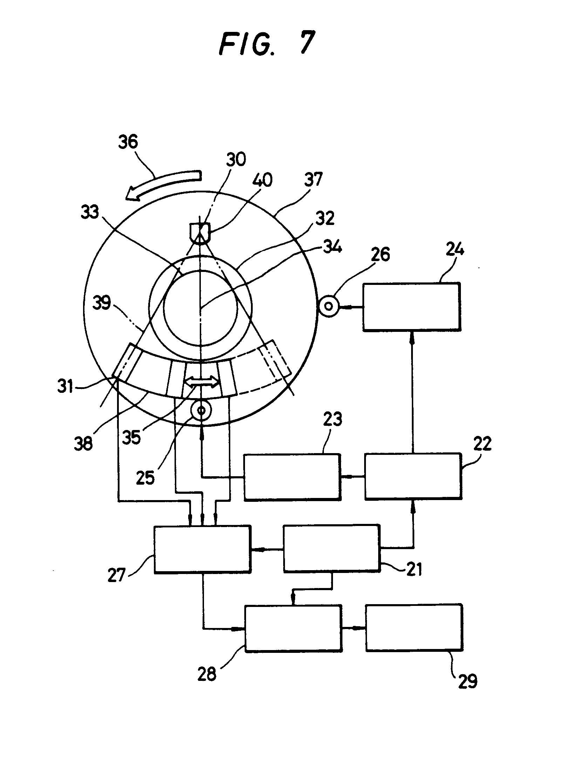

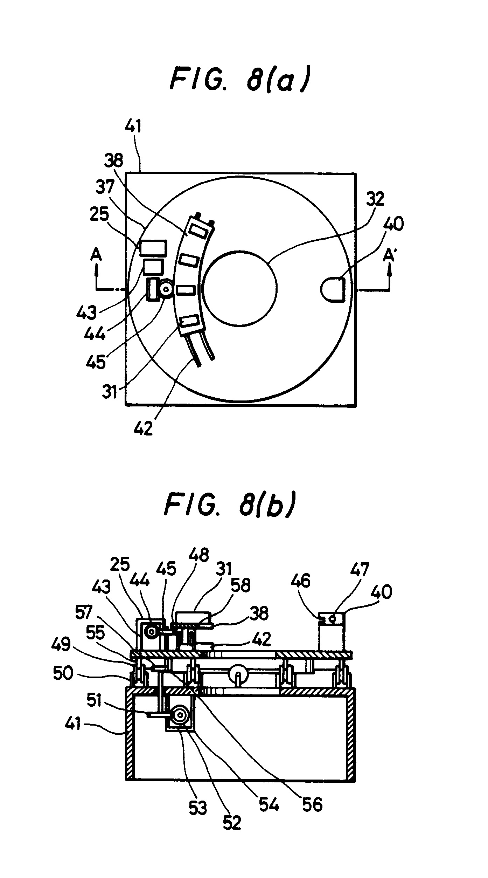

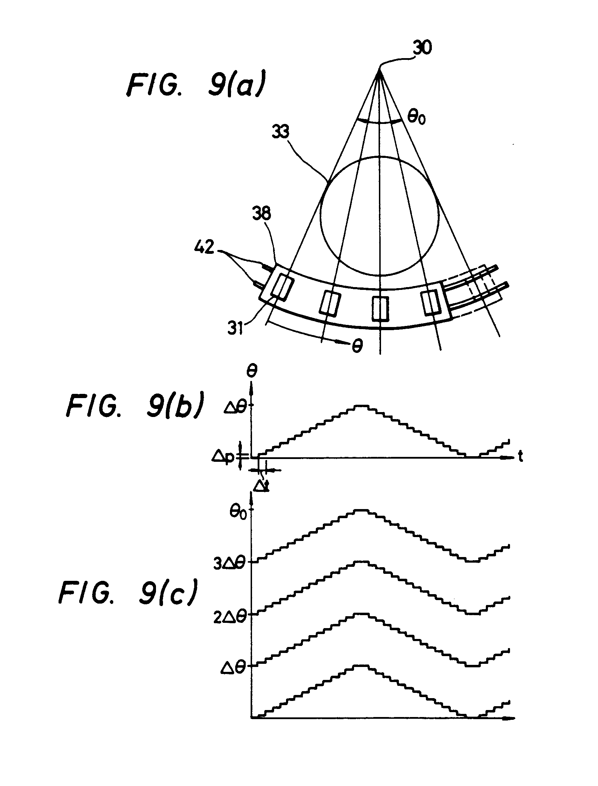

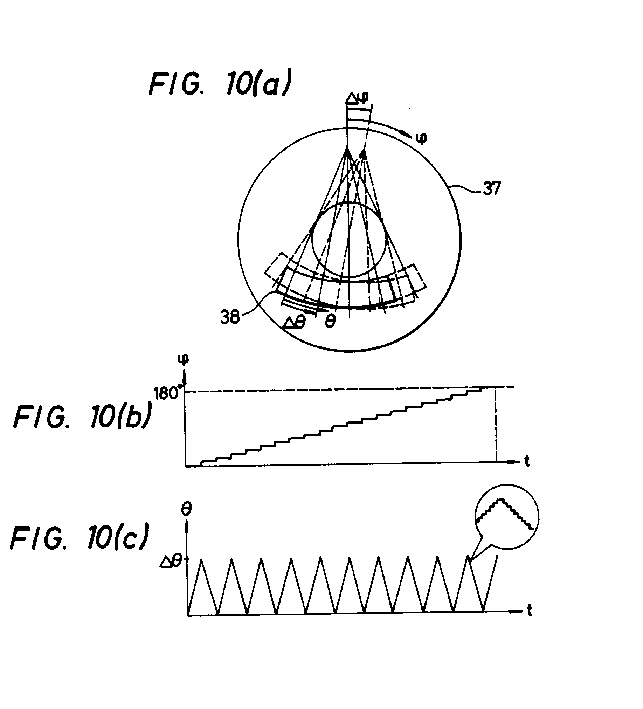

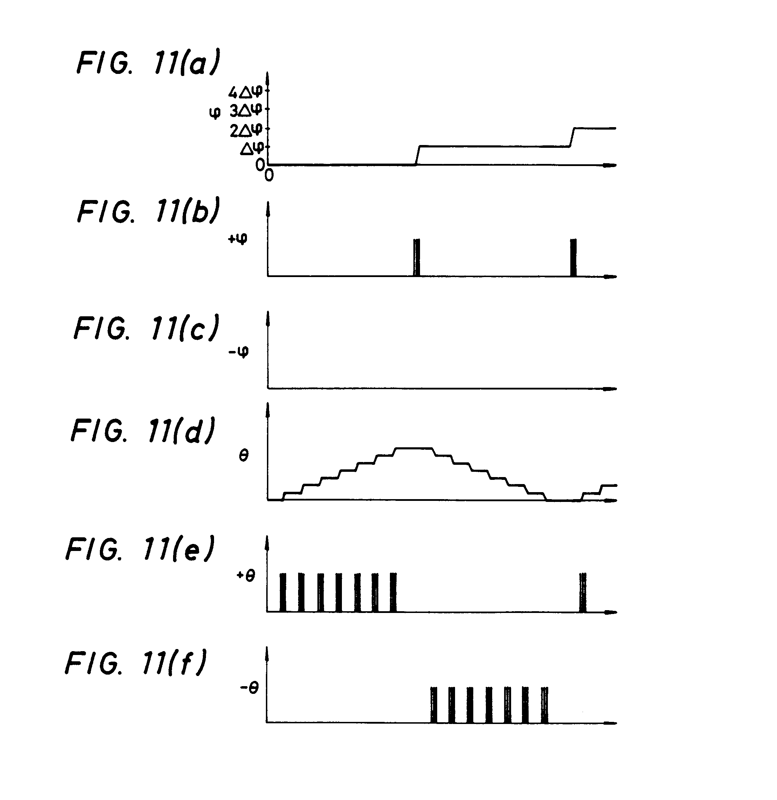

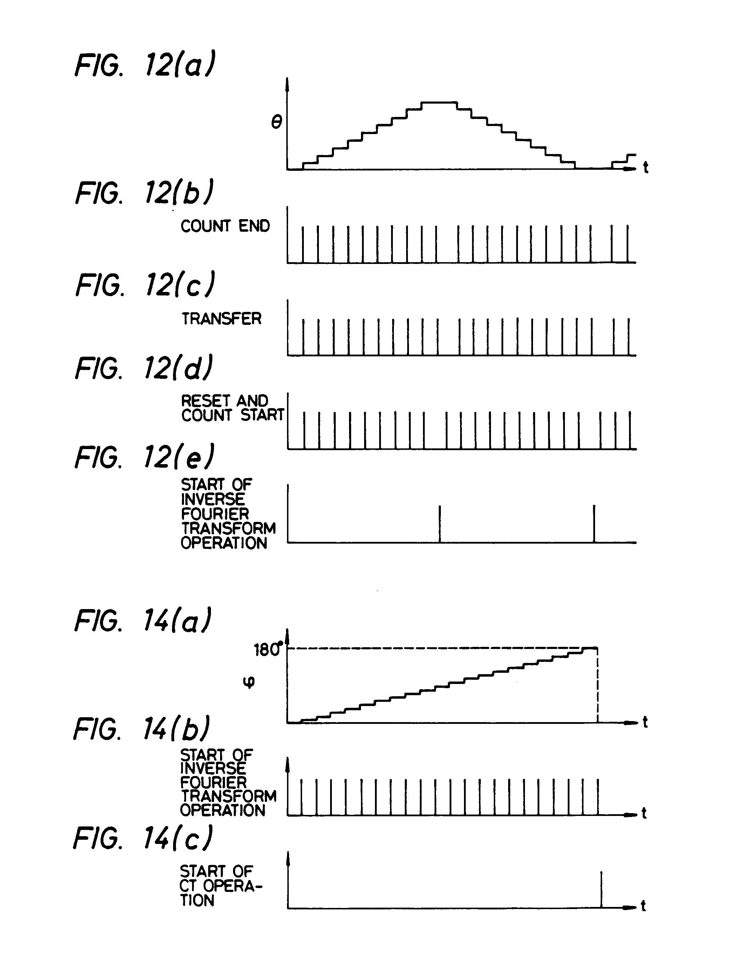

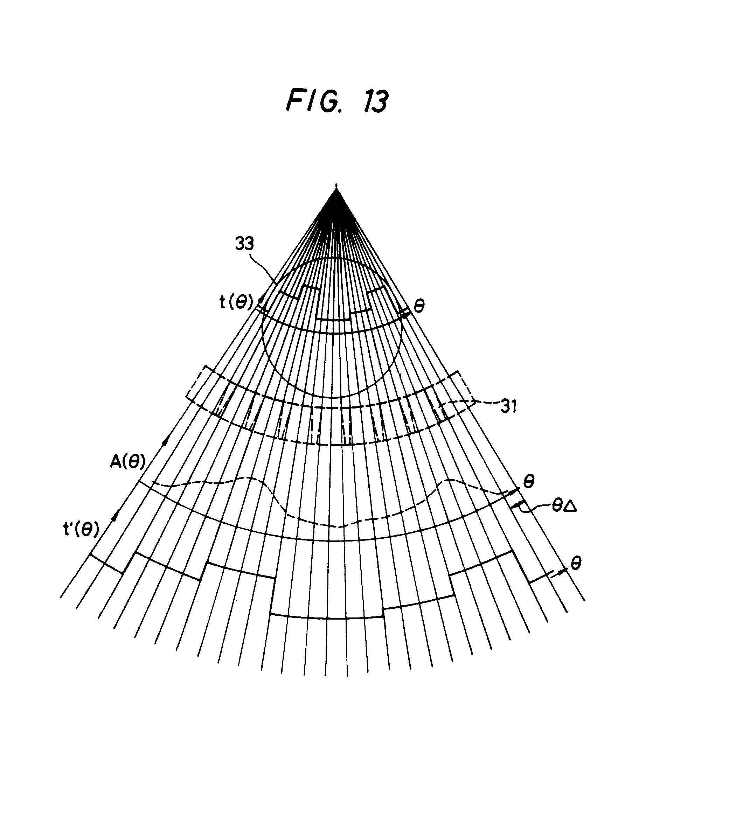

Fig. 1 is a plan view showing a scanning method in which the detector is scanned in a swinging manner around a radiation source; Fig. 2 is a plan view showing a scanning method in which a collimator is fitted to the radiation source in the method of Fig. 1; Fig. 3 is a plan view showing a scanning method in which a plurality of detectors are scanned in a swinging manner around the radiation source; Fig. 4 is a plan view showing a scanning method in which detectors are scanned in such a manner that detection ranges of radiation overlap each other; Fig. 5 shows the distribution of detection sensitivity of the detector; Fig. 6 shows procedures for obtaining a resolution as high as possible by a detector having a definite size; Fig. 7 shows an entire construction of an embodiment of the present invention; Figure 8 shows a structure of a radiation measuring apparatus to which the present invention is applied; Figure 9 is a time chart showing the respective positions of the detectors in swing scanning; Figure 10 is a time chart showing the relationship in time between swing scanning and rotational scanning; Figure 11 is a time chart of control signals for executing swing scanning and rotational scanning; Figure 12 is a time chart of a control signal for a radiation counter; Figure 13 shows an inverse Fourier transform operation processing in the swing scanning; and Figure 14 is a time chart of an image processing operation control signal.

## DESCRIPTION OF THE PREFERRED EMBODIMENT

The principle of the present invention will be described before the description of the embodiment with reference to drawings. To begin with, the disadvantages of the conventional way of taking the data on transmitted radioactive rays (this S-R system) will be summed up.

(1) It is impossible to arrange a number of radiation detectors closely to each other, and it is difficult to make the detectors small. Thus, some discreteness in data is inevitable.

(2) The axes of the detectors and of the radiation source are not the same; therefore, the thickness of detected beams is diverse.

The present invention has been designed for eliminating the aforesaid two faults.

Figure 1 shows an example in which one detector 2 opposed to a radiation source 12 and matched in the axis with the latter is subjected to swing scanning 13 conducted around the radiation source 12. A radiation beam 14 from the radiation source 12 is detected by the detector 2 subjected to said swing scanning 13 through every angle $\Delta\theta$ (fixed). If the radiation from the radiation source 12 were unidirectional, it would be necessary to turn the radiation source 12 through every angle $\Delta\theta$. In practice, however, it is unnecessary to turn the source in such a manner, since the radiation source 12 is generally isotropic in the direction of radiation except for an X-ray tube 3. The exposure to the radiation can be reduced, by providing the radiation source 12 with a collimator 15 as shown in Figure 2 and by scanning through every angle $\Delta\theta$ in synchronization with the detector 2.

When the swing scanning 13 is conducted around the radiation source 12 by using the radiation source 12, the collimator 15 and the detector 2 constructed as stated above, the radiation beam 14 is transmitted on an axis connecting the detector 2 and the radiation source 12, and the distance between the detector 2 and the radiation source 12 is fixed irrespective of the position of detection. Consequently, the aforesaid problem (2) of the diversity in the thickness of the detected beam can be settled.

However, the example of Fig. 1 in which one detector 2 is employed requires $n$ times more time for radiographing than in the case in which $n$ detectors 2 are employed, and thus said example is accompanied by the disadvantage that high-speed radiography can not be conducted. In this connection, a case in which a plurality of detectors 2 are employed will be shown in the following.

Figure 3 shows an example in which three detectors 2 are arranged on a circular-arc at an interval of an angle $\theta$ around the radiation source 12. If the angle $\theta$ is set to be twice as large as the angle $\Delta\theta$, the time required for radiographing the same region can be reduced to 1/3 of that in the case of Fig. 1. Although scanning through every $\Delta\theta$ is shown in Fig. 3, the angle $\Delta\theta$ is divided into $m$ minute segments in practice as shown in Figure 4, and radiographing is conducted by making the detectors 2 overlap. In other words, the detection of radiation is executed along a scanning line 16 at an angular pitch of $\Delta\theta/m$. Such execution of scanning makes it possible to avoid the discreteness of the detected data, and thus the aforesaid problem (1) can be settled.

An examination of the size of one detector 2 makes it clear that detected data such as those obtained by a delta function with respect to a position X is satisfactory for the present purpose. When expressed by a formula, they can be represented in the following equation.

$$a(0) = a_0 \int_{-\frac{X_0}{2}}^{\frac{X_0}{2}} \eta(X) r(X) \delta(X) dX = \eta(0) t(0) \quad \dots\dots\dots\dots \quad (1)$$

where $\eta(X)$ denotes the distribution of the detection sensitivity of the detector 2 and r(X) the distribution of the radiation transmission of a object 17, while $\delta(X)$ denotes a delta function and a (0) the detected intensity of radiation at $X = 0$. A value $a_0$ is a proportion constant, and $X_0$ denotes the aperture of the detector 2. The efficiency of detection is generally reduced extremely as the size $X_0$ of the aperture of the detector 2 is decreased, and therefore, the size $X_0$ of the aperture can not be decreased. When the size $X_0$ of the aperture of the detector 2 is enlarged, a detection region is expanded, and thus the detected amount a(0) of radiation at $X = 0$ is given in practice by the following equation as shown in Figure 5.

$$a(0) = a_0 \int_{-\frac{X_0}{2}}^{\frac{X_0}{2}} \eta(X) r(X) dx \quad \dots\dots\dots\dots\dots\dots \quad (2)$$

Accordingly, necessary information r(0) is obscured by a value a(0), and thus r(0) can not be extracted without any modification. When the equation (2) is rewritten with respect to X, the following equation is obtained.

$$a(X) = a_0 \int_{-\frac{X_0}{2}}^{\frac{X_0}{2}} \eta(\tau) r(X-\tau) d\tau \quad \dots\dots\dots\dots\dots \quad (3)$$

This is a convolution of the distribution $\eta(X)$ of the sensitivity of the detector 2 and the distribution r(X) of the radiation transmission of the object 17. The distribution $\eta(X)$ of the sensitivity of radiation detection and the detected intensity a(X) of radiation are the two amounts which can be observed solely according to the equation (3). Therefore, when Fourier-transformed values of the distribution $\eta(X)$ of the sensitivity of radiation detection, the distribution a(X) of the detected intensity of radiations and the unknown distribution r(X) of the radiation transmission of the object 17 are denoted by $H(\omega)$, $A(\omega)$ and $R(\omega)$ respectively, the equation (3) can be rewritten as follows:

$$A(\omega) = a_0 H(\omega) R(\omega) \quad (4)$$

Accordingly, the following equation can be obtained from the equation (4).

$$R(\omega) = \frac{A(\omega)}{A_0 H(\omega)} \quad (5)$$

Thus, the unknown distribution r(X) of the radiation transmission of the object 17 can be determined from the following formula, which is obtained by applying the inverse Fourier transformation $F^{-1}$ to the equation (5).

$$r(X) = F^{-1}[R(\omega)]$$
$$= F^{-1}\left[\frac{A(\omega)}{a_0 H(\omega)}\right] \quad \dots\dots\dots\dots \quad (6)$$

The above-stated procedures are illustrated in Figure 6. The object 17 having the distribution r(X) of the radiation transmission and the distribution t(X) of the thickness is held between the radiation source 12 and the detector 2, a translational scanning 18 is applied thereto, and thereby the distribution A(X) of the intensity thereof is measured. When the distribution $\eta(X)$ of the sensitivity of the radiation detection of the detector 2 is known on the occasion, an estimated value r'(X) of the distribution r(X) of the radiation transmission can be determined from the Fourier-transformed value $H(\omega)$ of $\eta(X)$ and the Fourier-transformed value $A(\omega)$ of the distribution A(X) of the detected intensity, according to the formula (6), and an estimated value t'(X) of the distribution t(X) of the thickness can be determined therefrom. The values r'(X) and t'(X) are approximate because

4

the distribution a(X) of the intensity detected by the detector 2 contains a noise component and thus the equation (3) is not valid in the strict sense of an equation.

The above-described procedures improve resolution even when the detector 2 has a large aperture. The application of these procedures to the method of detecting radiation shown in Figs. 3 and 4 enables the attainment of a sectional image of high resolution from radiations, and thus all the disadvantages of the S-R system of the fourth generation can be settled.

An embodiment of the present invention will be described hereunder with reference to Figure 7. Figure 7 shows a construction of the whole of an apparatus formed by applying a radiation-measuring method of the present invention to a gamma-ray CT apparatus. The main control unit 21 for controlling the whole of said apparatus and taking in the data on the radiation transmission is connected to a mechanical scan control unit 22, a radiation counter 27 and an image processing unit 28, and supplies said mechanical scan control unit 22 with a position control signal for scanning a radiation measuring table 37 and a detector scanning stage 38, supplying the radiation counter 27 with a count end signal, a reset signal and a count start signal, and further supplying the image processing unit 28 with a take-in signal for taking in the result of a count after the count end signal is sent to the radiation counter 27, and a process start signal. The mechanical scan control unit 22 is connected to the main control unit 21, a detector scan control unit 23 and a rotational scan control unit 24, and divides the position control signal from the main control unit 21 into a detector scan signal and a rotational scan signal, supplying the detector scan control unit 23 and the rotational scan control unit 24 with the detector scan signal and the rotational scan signal respectively. The detector scan control unit 23 is connected to the mechanical scan control unit 22 and a pulse motor 25 for detector scanning, receiving the detector scan control signal from the mechanical scan control unit 22, converting same into a signal for driving the pulse motor, and sending it to said pulse motor 25. The rotational scan control unit 24 is connected to the mechanical scan control unit 22 and a pulse motor 26 for rotational scanning, receiving the rotational scan control signal from the mechanical scan control unit 22, converting same into a signal for driving the pulse motor, and sending it to the pulse motor 26. The pulse motor 26 for rotation scanning is connected to the rotational scan control unit 24, and rotates according to the pulse motor driving signal sent from the rotational scan control unit 24. The power of this pulse motor is transmitted to the radiation measuring table 37 connected thereto mechanically to make it conduct a rotating movement 36 arund an axis 34 of rotation. The radiation measuring table 37 is provided with a radiation source 40, the detector scanning stage 38 fitted with a plurality of radiation detectors 31, and the pulse motor 25 for detector scanning. This table is connected mechanically with the pulse motor 26 for rotational scanning, and is rotated around the axis 34 of rotation by the power of said pulse motor 26. Moreover, the radiation measuring table 37 is provided with an aperture 32 made therein so that it contains an inspection region 33. The detector scan control unit 23 is connected to the mechanical scan control unit 22 and the pulse motor 25 for detector scanning, receiving the detector scan control signal from the mechanical scan control unit 22, converting same into the pulse motor driving signal, and sending it to the pulse motor 25. This pulse motor 25 is provided on the radiation measuring table 37 and connected to the detector scan control unit 23, and rotates according to the pulse motor driving signal from the detector scan control unit 23. The power of said pulse motor 25 is transmitted to the detector scanning stage 38 to make it conduct a swinging movement 35 around an axis of swinging 30. On the detector scanning stage 38 three detectors 31 are provided so that the detecting surfaces thereof are directed to the axis 30 of swinging and that straight lines (radiation beam paths 39) connecting the axis 30 of swinging with the radiation detectors 31 cover the entire inspection region 33. The radiation source 40 is provided in alignment with the axis 30 of swinging. Radioactive rays transmitted through the inspection region 33 are detected by the three radiation detectors 31 according to the rotating mouvement 36 of the radiation measuring table 37 and the swinging movement 35 of the detector scanning stage 38. The radiation detectors 31 are connected to the radiation counter 27 respectively, and supply the radiation counter 27 with data on detected radiations. The counter 27 is connected to the radiation detectors 31, the main control unit 21 and the image processing unit 28, receiving the radiation count end signal, the reset signal and the count start signal from the main control unit 21, and sending data on counted radiations to the image processing unit 28 based on the radiation count end signal. The image processing unit 28, connected to the main control unit 21, the radiation counter 27 and an image display unit 29, takes in the data on counted radiation from the radiation counter 27 based on a signal for taking in data on counted radiation, which is sent from the main control unit 21, and starts an image processing for image display based on an image processing start signal sent from the main control unit 21. After ending an operation of prescribed data, the image processing unit 28 sends the result of the operation to the image display unit 29. A signal for ending the operation of prescribed data is supplied from the main control unit 21. The image display unit 29, connected to the image processing unit 28, receives the result of the operation sent thereto after the end of the operation by the image processing unit 28, converts same into an image signal and thereby execute the image display. The following is a description on the functions of the above-described units and components, which can be divided into two functions - mechanical scanning and signal processing.

First, a description will be made on a section of mechanical scanning. Figure 8 shows a structure of a radiation measuring apparatus which is an application of the present invention. The entire apparatus is installed on a frame 41, and the swing scanning and the rotational scanning are performed by the pulse motors. The frame 41 is fitted with a plurality of support wheels 49 by means of bearings 50 so that the rotating motion of the radiation measuring table 37 can be performed smoothly. These support wheels 49 rotate on rails 55 provided under the radiation measuring table 37, so as to rotate this table freely. Power to drive the radiation measuring table 37 is obtained from a pulse motor 54, which rotates in a prescribed manner based on a signal from the rotational scan control unit 24. This pulse motor 54 is connected to a reduction gear 53 for rotational scanning so that it can transmit a power thereto. The reduction gear 53 for rotational scanning is connected to a worm gear 52 for rotational scanning and can transmit a power thereto. This worm gear 52 for rotational scanning is combined with a gear 51 for rotational scanning so that the axes of rotational powers are made to intersect perpendicularly to each other, thereby the rotational power thereof being transmitted to a rotational gear 57. The rotational gear 57 engages with a gear 56 provided under the radiation measuring table 37 and thereby can transmit the power of the pulse motor 54 to this table 37. Next, a description will be made of the mechanism of the detector scanning stage 38. The detector scanning stage 38 is provided beneath with a plurality of wheels 58 for swing scanning, and these wheels 58 ride on rails 42 laid in the shape of an arc around the radiation source 40 on the radiation measuring table 37, so that the stage can conduct a swing movement around the radiation source 40. A power for this circular-arc movement is given by the pulse motor 25, which rotates in a prescribed manner based on a signal from the detector scan control unit 23. This pulse motor 25 is connected to a reduction gear 43 for swing scanning and can transmit a power of rotation thereto. The reduction gear 43 for swing scanning is connected to a worm gear 44 for swing scanning and can transmit the power thereto. This worm gear 44 for swing scanning is combined with a gear 45 for swing scanning to make the axes of the rotational power intersect perpendicularly to each other so that the rotational power thereof can be transmitted to the gear 45 for swing scanning. This gear 45 engages a circular-arc shaped gear 48 provided in the lower part of the detector scanning stage 38 and thereby can transmit the power of the pulse motor 25 to the detector scanning stage 38. The foregoing is the description on the mechanical scanning units and components.

Next, a description will be made on signals for controlling these mechanical scanning units and components. Figure 9 shows the respective positional angle of four radiation detectors 31 in the case when swing scanning is performed by these radiation detectors 31. When an angular aperture containing an inspection region 33 with the swinging axis 30 of the detector scanning stage as the center is $\theta_0$ and the whole of this angular aperture $\theta_0$ is covered by using the four radiation detectors 31, the scanning angle $\Delta\theta$ of the detector scanning stage 38 is $\theta_0/4$. It is found that a scanning angle $\Delta\theta$, required when $m$ detectors are employed, is given by $\theta_0/m$. In this figure, symbol $\Delta t$ denotes time required for measuring radiation. The time required for one swing scanning is given by

$$\Delta t \times \frac{\Delta\theta}{\Delta}$$

when the swing scanning of $\Delta\theta$ is conducted at a pitch $\Delta P$. Figure 10 shows the relationship between swing scanning and rotational scanning, in which swing scanning angle $\theta$ and rotational scanning angle $\psi$ are used. This means that the radiation measuring table 37 is rotated $\Delta\psi$ every time when the swing scanning of $\Delta\theta$ is ended, and then a swing scanning of $-\Delta\theta$ is started. The foregoing is the description on the positional relationship between the radiation measuring table 37 and the detector scanning stage 38, and next a description will be made on signals supplied from the rotational scan control unit 24 and the detector scan control unit 23 to the respective pulse motors 54 and 25. Figure 11 shows positions of these units and control signals therefor. The figure shows a position signal for the radiation measuring table 37, pulse signals (forward and backward signals from above downward) given to the pulse motor 54 for driving the radiation measuring table 37, a position signal for the detector scanning stage 38, and pulse signals (forward and backward signals from above downward) given to the pulse motor 25 for driving the detector scanning stage 38. Such a train of pulse signals are supplied from the main control unit 21 through the mechanical scan control unit 22. Next, a description will be made of a section in which radiation count signals are supplied from a plurality of radiation detectors 31 provided on the detector scanning stage 38 to the radiation counter 27 and are not transferred therefrom to the image processing unit 28. The detector scanning stage 38 moves at the pitch of $\Delta P$, stops for the time $\Delta t$ and measures radiations during this time, while the radiation counter 27 is supplied with a count end signal, a transfer signal for transferring the number of counts to the image processing unit 28 and reset and count start signals from the main control unit 21. Time charts of these signals are shown in Figure 12. When the swing scanning of $\Delta\theta$ is ended, an inverse Fourier transform operation start signal is sent from the main control unit 21 to the image processing unit 28, whereby an inverse Fourier transform operation is started. Figure 13 shows radiation count signals in which the symbol $A(\theta)$ denotes the distribution of radiation transmission which is obtained from the detection of the distribution t(θ) of radiation transmission within the inspection region 33 by the radiation detectors 31. Owing to the distribution of the detection sensitivity of the detectors 31, the former distribution is obscure in comparison with the latter distribution. Therefore, the former distribution is modified into data as shown in t'(θ) by the inverse Fourier transform operation described previously, and the data thus obtained are stored in a memory in the image processing unit 28. Time charts of the position signal of the radiation measuring table 37, the inverse Fourier transform operation start signal and a CT operation start signal are shown in Figure 14.

In the above-described embodiment, inverse Fourier transform operation is conducted when each swing scanning is ended. The same effect can be produced also when said processing is conducted after all the mechanical scannings are ended.

## Claims

1. A cross-sectional radiography imaging apparatus comprising a radiation source (40), a plurality of radiation detectors (31) for detecting radiant rays emitted from said radiation source (40) and transmitted through an object to be measured and for providing output signals indicative thereof said radiation detectors (31) being respectively arranged at predetermined spaced intervals from each other along a portion of a circumference of a circle centered on said radiation source so that a respective incident radiation enters each detector at a right angle thereto and being connected together for simultaneous movement, driving means (26, 49-51) for moving said radiation source simultaneously with said radiation detector, means (27, 28) for computing cross-sectional imaging information on said object in response to the output signals from said radiation detectors (31), means (29) for displaying a cross-sectional image of the object in accordance with the output of said computing means (27, 28), and detector driving means (25, 42-45, 48, 58) which additionally move said radiation detectors at a minute angular pitch on a part of the circumference of the circle centered on said radiation source,

characterized in that said detector driving means moves said radiation detectors at the minute angular pitch corresponding to a distance less than the width of one of the radiation detectors and said computing means (27, 28) comprises a computer (28) having a memory means storing an operation program in which the computation consists of converting signals a(x) detected by each radiation detector (31) into a spectrum of a spacial frequency A(ω)

with regard to a one-dimensional scanning position of each said radiation detector (31), dividing A(ω) by the Fourier transformed value H(ω) of the distribution η(x) of the sensitivity of the detector, multiplied with a proportionality constant $a_o$ and inversely converting this quotient into a spectrum r(x) of real coordinate transmission with regard to said scanning position of the radiation detector, and a section of the object to be measured is displayed by using signals which signify said inversely-converted spectrum outputted from said computer.

## Patentansprüche

1. Querschnittsradiosgraphische Vorrichtung mit einer Strahlungsquelle (40), mehreren Strahlungsempfängern (31) zum Empfang einer von der Strahlungsquelle (40) ausgesandten und durch einen zu messenden Gegenstand durchgelassenen Strahlung und zur Lieferung entsprechender Ausgangssignale, wobei die Strahlungsempfänger (31) jeweils in vorbestimmten Abstandsintervallen voneinander entlang eines Teils eines auf die Strahlungsquelle zentrierten Kreisumfangs angeordnet sind, so daß eine jeweilige einfallende Strahlung in jeden Empfänger in einem dazu rechten Winkel eintritt und wobei die Strahlungsempfänger (31) zur gleichzeitigen Bewegung miteinander verbunden sind, einer Antriebseinheit (26, 49 - 51) zur Bewegung der Strahlungsquelle gleichzeitig mit den Strahlungs empfängern, einer Einheit (27, 28) zur Berechnung der Querschnitts-Bildinformation auf dem Gegenstand abhängig von Ausgangssignalen der Strahlungsempfänger (31), einer Einheit (29) zur Anzeige eines Querschnittbildes des Gegenstands entsprechend den Ausgangssignalen der Rechnereinheit (27, 28) und einer Empfängerantriebseinrichtung (25, 42 - 45, 48, 58), die zusätzlich die Strahlungsempfänger um einen sehr kleinen Teilungswinkel auf einem Teil eines auf die Strahlungsquelle zentrierten Kreisumfangs bewegt,

**dadurch gekennzeichnet,**

daß die Empfängerantriebseinrichtung die Strahlungsempfänger um einen sehr kleinen Teilungswinkel bewegt, der einem Abstand entspricht, der geringer ist als die Breite eines Strahlungsempfängers und daß die Rechnereinheit (27, 28) einen Rechner (28) mit einer Speichereinheit aufweist, die ein Rechenprogramm speichert, in dem die Berechnung besteht aus der Umwandlung der von jedem Strahlungsempfänger (31) aufgenommenen Signale in ein räumliches Frequenzspektrum $A(\omega)$ in bezug auf eine eindimensionale Abtastposition jedes Strahlungsempfängers (31), Division von $A(\omega)$ durch den Fourier-transformierten Wert $(H(\omega))$ der Empfindlichkeitsverteilung $\eta(x)$ des Empfängers, multipliziert mit einer Proportionalitätskonstante $a_o$ und inverse Umwandlung dieses Quotienten in ein Übertragungsspektrum $r(x)$ realer Koordinaten in bezug auf die Abtastposition des Strahlungsempfängers, und daß ein Schnitt des zu messenden Gegenstandes angezeigt wird durch Verwendung von Signalen, die das invers umgewandelte Spektrum anzeigen, welches vom Rechner ausgegeben wird.

**Revendications**

1. Dispositif de formation d'images radiographiques de coupes transversales, comprenant une source de rayonnement (40), une pluralité de détecteurs de rayonnement (31) servant à détecter des rayons émis par ladite source de rayonnement (40) et transmis à travers un objet devant être mesuré, et servant à fournir des signaux de sortie indicatifs de cet objet, lesdits détecteurs de rayonnement (31) étant disposés respectivement à des intervalles prédéterminés les uns des autres le long d'une partie d'une circonférence d'un cercle centré sur ladite source de rayonnement de sorte qu'un rayonnement incident respectif pénètre dans chaque détecteur perpendiculaire à ce dernier, et raccordés entre eux de manière à se déplacer simultanément, des moyens d'entraînement (26,46-51) servant à déplacer ladite source de rayonnement en même temps que lesdits détecteurs de rayonnement, des moyens (27,28) servant à calculer l'information de formation d'images de coupes transversales concernant ledit objet en réponse aux signaux de sortie délivrés par lesdits détecteurs de rayonnement (31), des moyens (29) pour afficher une image d'une coupe transversale de l'objet conformément au signal de sortie desdits moyens de calcul (27,28), et des moyens (25,42-45,48,58) d'entraînement des détecteurs, qui déplacent en outre lesdits détecteurs de rayonnement selon un faible pas angulaire sur une partie de la circonférence du cercle centré sur ladite source de rayonnement, caractérisé en ce que lesdits moyens d'entraînements des détecteurs déplacent lesdits détecteurs de rayonnement selon le faible pas angulaire correspondant à une distance inférieure à la largeur de l'un des détecteurs de rayonnement et que lesdits moyens de calcul (27,28) comprennent un ordinateur (28) possédant des moyens de mémoire mémorisant un programme d'opérations et dans lequel le calcul consiste à convertir les signaux $a(x)$ détectés par chaque détecteur de rayonnement (31) en un spectre d'une fréquence spatiale $A(\omega)$ en rapport avec une position de balayage unidimensionnel de chaque détecteur de rayonnement (31), diviser $A(\omega)$ par la valeur $H(\omega)$ de la transformée de Fourier de la distribution $\eta(x)$ de la sensibilité du détecteur, multipliée par une constante de proportionnalité ao et réaliser la conversion inverse de ce quotient en un spectre $r(x)$ de transmission de coordonnées réelles en rapport avec ladite position de balayage du détecteur de rayonnement, une coupe de l'objet à mesurer étant affichée moyennant l'utilisation de signaux signifiant que ledit spectre obtenu par conversion inverse est délivré par ledit ordinateur.

# FIG. 1

$\triangle\theta = const$

# FIG. 2

$\triangle\theta = const$

EP 0 188 782 B2

FIG. 3

FIG. 4

FIG. 5(a)  FIG. 5(b)

FIG. 5(c)  FIG. 5(d)  FIG. 5(e)

$$a = a_0 \int p \, dx$$

$$p(x) = \eta(x) \cdot r(x)$$

FIG. 6(a)

FIG. 6(c)

$$\frac{1}{2\eta}\int \frac{A(\omega)}{a_0 H(\omega)} e^{i\omega x} dx$$

FIG. 6(b)

FIG. 7

## FIG. 8(a)

## FIG. 8(b)

FIG. 9(a)

FIG. 9(b)

FIG. 9(c)

FIG. 10(a)

FIG. 10(b)

FIG. 10(c)

FIG. 11(a)

FIG. 11(b)

FIG. 11(c)

FIG. 11(d)

FIG. 11(e)

FIG. 11(f)

**FIG. 12(a)**

θ

**FIG. 12(b)**

COUNT END

**FIG. 12(c)**

TRANSFER

**FIG. 12(d)**

RESET AND
COUNT START

**FIG. 12(e)**

START OF
INVERSE
FOURIER
TRANSFORM
OPERATION

**FIG. 14(a)**

180°

φ

**FIG. 14(b)**

START OF
INVERSE
FOURIER
TRANSFORM
OPERATION

**FIG. 14(c)**

START OF
CT OPERA-
TION

FIG. 13